# EUROPEAN PATENT SPECIFICATION

(11) **EP 0 704 527 B1**
(45) Date of publication and mention of the grant of the patent: **07.05.2003**
(21) Application number: 95112210.0
(22) Date of filing: 03.08.1995
(51) Int. Cl.: C12N 15/17, C12N 1/15, C12N 15/62, C07K 14/62

(54) **DNA sequences encoding biosynthetic insulin precursors and process for preparation of insulin**
Für biosynthetische Insulinvorstufen kodierende DNA-Sequenzen und Verfahren zur Herstellung von Insulin
Séquences d'ADN codants les précurseurs d'insuline et procédé pour la préparation d'insuline

(30) Priority: 05.08.1994 HR 940432
(43) Date of publication of application: 03.04.1996
(73) Proprietor: Pliva, farmaceutska industrija, dionicko drustvo, 10000 Zagreb (HR)
(72) Inventor: Mestric, Silvija, HR-41000 Zagreb (HR); Punt, Peter J., NL-2642 AX Pijnacker (NL); Valinger, Radovan, H-41000 Zagreb (HR); van den Hondel, Cees A.M.J.J., NL-2804 PZ Gouda (NL)
(74) Representative: von Füner, Alexander, Prof.h.c. Dr.

(56) References cited:
- EP-A- 0 196 056
- EP-A- 0 249 350
- EP-A- 0 324 274
- WO-A-92/11378
- WO-A-95/22614
- BIOCHEMICAL SOCIETY TRANSACTIONS, vol. 19, no. 3, August 1991, pages 681-685, XP000676669 R.M. BERKA ET AL.: "The decelopment of Aspergillus niger var. awamori as a host for the expression and secretion of heterologous gene products"

## Description

This invention relates to DNA sequences encoding novel biosynthetic insulin precursors and to preparation of insulin by culturing transformed cells comprising such DNA sequences.

Insulin is a hormone, whose primary role is to control the transport of glucose from the bloodstream into cells where it is metabolized.

Insulin consists of two chains of amino acids: A-chain consisting of 21 amino acids and B-chain consisting of 30 amino acids, the chains being linked with each other by two disulfide bridges. A third disulfide bridge is to be found within the A-chain.

The primary form of insulin molecule is synthesized as a nascent polypeptide chain named preproinsulin,which consists of a signal peptide and proinsulin. The signal peptide directs the nascent chain across the endoplasmic reticulum. Proinsulin is produced from preproinsulin in the lumen area of endoplasmic reticulum as soon as the polypeptide chain crosses the membrane. Proinsulin encompasses a polypeptide chain comprising a chain of 35 amino acids which is not present in the insulin. This connecting peptide or C-peptide links the C-end of the B-chain to the N-end of the A-chain of the future insulin molecule. From the lumen of endoplasmic reticulum proinsulin is routed to the Golgi apparatus where the proteolysis of connecting peptide starts. The connecting or C-peptide is cut out by an enzymatic system similar to trypsin/carboxypeptidase B system, which acts at the two consecutive basic amino acids sequence and insulin is produced (Steiner et al., J. Cell Biol. 24, (1984) 121-130).

The importance of the C-peptide is that it links the B- and A-chains so as to allow a proper formation of disulfide bridges in the insulin molecule (Bell et aL, Nature 284 (1980) 26-32) or that it directs the trypsin-like converting enzyme to process proinsulin at the site with two basic amino acids (Thim et al., PNAS, 83 (1986) 6766-6770).

The production of biosynthetic human insulin has been reported mainly in the bacterium *Escherichia coli* and the yeast *Saccharomyces cerevisiae.* It is common for all of them that DNA sequence encodes either for the entire proinsulin, a modified part of proinsulin or separately for the A- and B-chains.

In the bacterium *E. coli* insulin has been produced either via separate A- and B-chains (Chance et al., Diabetes Care 4 (1982) 147-154) or as proinsulin, EP 55945.

In the yeast *S. cerevisiae* insulin has been produced via proinsulin, yet with a very low yield, EP-A-121 884. Various modifications of C-peptide improve the yield and the proper formation of the insulin molecule in the yeast.

Insulin precursors of formula B-X-A, wherein B and A represent the B- and A-chains and X represents peptide comprising from 2 to 35 amino acids, are described in DK-A-5284/87.

Insulin precursors of formula B-X-Y-A, wherein B and A represent the B- and A-chains and X and Y each represent lysine or arginine, are disclosed in EP-A-195 691.

In EP-A-163 529 insulin precursors containing the peptide chain B(1-29)-A(1-21) linked with a short peptide having 2 to 8 amino acids are described. Modifications of des-B(30) insulin precursors of formula B(1-29)-X₁-X₂-Y₂-Y₁-A(1-21) are described in EP-A-347 845.

In EP 249 350 glucoamylase promoter and signal sequence for secretion of proteins in fungi as hosts are described. As an example, an expressed proinsulin trimer was also inserted into such an expression vector.

Many polypeptides for therapeutic or diagnostic use, e.g. erythropoietin, tPA and factor VIII, have been N-glycosylated (Parekh et al., TIBTECH 7 (1989) 117-121).

There has also been described a triply increased yield of bovine chymosin (glycochymosin) in the fungus *A. niger* var. *awamori* achieved by the introduction of a N-glycosylation consensus site into a coding sequence. By a fusion of prochymosin and glucoamylase gene even a higher yield was achieved, but by a fusion of glycochymosin to glucoamylase no further increase of the yield was achieved (Berka et al., Biochem. Soc. Trans, 19(1991) 681-685).

In the case of aspartic proteinase (renin), which is of microbial origin and serves as a chymosin substitute, it has been observed that hyperglycosylation may reduce the enzyme activity if it occurs at a critical site in the molecule. (Berka et al., Biochem. Soc. Trans. 19 (1991) 681-685; Aikawa et al., J.Biol.Chem. 265 (1990) 13955-13959).

A mutation of glycosylated asparagine in the coding region of renin resulted in a significant reduction of secretion in yeast cells (Aikawa et al., J.Biol.Chem. 265 (1990) 13955-13959).

The object of the invention are DNA sequences encoding novel biosynthetic insulin precursors and the use of eucaryotic cells, particularly fungal cells for preparation of insulin.

The object has been achieved by means of a DNA sequence comprising encoding sequence for insulin precursors of the formula B-Pg-A, wherein B and A represent B- and A-chain respectively and Pg represents a modified C-peptide or any number of amino acids containing at least one N-glycosylation consensus-site. Pg links B- and A-chains by proteolytic processing signals or specific chemical cleavage sites.

The modification of proinsulin gene is preferred by creating N-glycosylation site AsnXSer. This can be achieved in a C-peptide sequence e.g. by changing the codon GCC for Ala-C-20 amino acid to the codon AAC for Asn.

The same principle can be applied to des-B(30)-proinsulin.

The invention is based on the finding that the yield of immunoreactive insulin, measured by RIA method, is much higher in transformants comprising DNA sequence of the formula B-Pg-A fused with protective protein than in transformants comprising a nonmodified DNA sequence of proinsulin fused with a protective protein. The cells transformed with a vector comprising the DNA sequence of nonfused proinsulin or nonfused modified proinsulin with N-glycosylation consensus site give a very low yield or none at all of immunoreactive insulin, measured by RIA method.

Therefore the introduction of N-glycosylation consensus site to a linker region which is not a part of a mature insulin molecule remarkably increases the expression in fungal cells which are transformed with the DNA sequence encoding such fused insulin precursors. Fused protein and proinsulin can either be processed in the host organism itself or removed by a later enzymatic or chemical cleavage.

The DNA sequence encoding the said modified insulin precursor can be prepared by an oligonucleotide synthesis of the entire DNA sequence in a combination with PCR or by *in vitro* mutagenesis. The DNA sequence can also be codon-optimized for expression in a suitable organism, preferably in a fungal strain.

The invention also relates to a process for the preparation of insulin by culturing suitable cells, preferably fungal cells, transformed with a vector comprising a DNA sequence encoding insulin precursor of the formula B-Pg-A in a suitable culture medium.

The expression and secretion of the peptide of the above formula can be achieved by using a vector in which the encoding sequence is regulated by a promoter, a signal sequence and a terminator functional in a suitable organism. For example, it can be p_{glaA} and a signal sequence of *glaA* gene or a part thereof suitable for expression in *Aspergillus sp*. (Cullen et al., Biotechnology, 5 (1987) 369-376). The expression and secretion of the peptide can also be achieved by using an expression vector in which DNA sequence encoding the insulin precursor of the above formula is fused to the gene of protective protein via a processing signal (Ward et al., Biotechnology 8 (1990) 435-440; Broekhuijsen et al., J. Biotech. 31 (1993) 135-145) or a chemical cleavage site.
**Fig. 1** shows a nucleotide sequence of both, the natural and the codon-optimized proinsulin gene and a corresponding amino acids sequence, using conventional abbreviations for nucleotides and amino acids.
**Fig. 2** describes two types of human proinsulin gene expression cassettes and schematically shows the human proinsulin gene and mutant gene comprising N-glycosylation consensus site.
**Fig. 3** explains the strategy of construction of human proinsulin gene and mutant gene comprising N-glycosylation consensus site for two types of expression cassettes via fragments synthesized separately by "overlapping oligonucleotides in combination with PCR".
**Fig. 4** shows 14 oligonucleotides designed for the synthesis of human proinsulin gene and mutant gene comprising N-glycosylation consensus site.
**Fig. 5** explains, on the example of fragment I synthesis, the novel approach that was used for the synthesis of human proinsulin gene by the use of overlapping, complementary oligonucleotides in combination with PCR.
**Fig. 6** schematically shows the synthesis of fragment IV (oligonucleotides are indicated by arrows only).
**Fig. 7** schematically shows the synthesis of fragment II, fragment III and fragment V (oligonucleotides are indicated by arrows only).
**Fig. 8** shows the construction of plasmids pPZG301, pPZG302, pPZG303, pPZG304, pPZG305.
**Fig. 9** shows the construction of plasmids pPZG311, pPZG312.
**Fig. 10** shows the construction of the filamentous fungal expression vectors for expression cassettes of type I.
**Fig. 11** shows the construction of the filamentous fungal expression vectors for expression cassettes of type II.
**Fig. 12** shows the sequence at the GlaG2 junction of *glaA* gene.

### Examples

### Example 1

### Synthesis of human proinsulin gene and mutant gene comprising N-glycosylation consensus site by overlapping oligonucleotides in combination with polymerase chain reaction (PCR)

### a) Methodology

All DNA manipulation experiments were made using standard molecular biology methods as described in Maniatis, T., et al., "Molecular Cloning, A Laboratory Manual", Cold Spring Harbor Laboratory, U.S.A. (1982).

All chemicals, enzymes and plasmids, unless otherwise stated, are available from various commercial sources. Buffers and reaction conditions for restrictive endonuclease digestions were used as recommended by manufacturer unless otherwise indicated.

Chemical synthesis of oligonucleotides was performed, upon order, by "ISOGEN Bioscience", Amsterdam, The Netherlands. DNA samples were supplied in a lyophilized form as sodium salts with free hydroxyl groups on 5' and 3' ends. Such DNA was dissolved in sterile MQ H₂O. The concentration of single-chain DNA oligonucleotides was determined by measuring absorbance at A₂₆₀. Samples of dissolved DNA-oligonucleotides were stored at -20°C.

The polymerase chain reaction, further indicated as PCR, was performed by Perkin Elmer Cetus (761 Main Ave., Norwalk CT 06859) apparatus, and GeneAmp™ DNA Amplification Reagent Kit with AmpliTaq™ Recombinant Taq DNA Polymerase. Concentrated solutions and reaction mixtures were prepared according to "Perkin Elmer Cetus Protocol for DNA Amplification" as recommended by the manufacturer, with the exception that in the reaction mixture there was no template DNA but only only oligonucleotides in a concentration of 100 pmol per reaction (100 µl). The temperature cycles profile for each PCR was: melting temperature 94°C/1 min, annealing temperature 40°C/1 min (for fragments I and IV) or 50°C/1 min (for fragments II, III and V), polymerization temperature 72°C/1 min. There were 25 cycles. After the last cycle, polymerization time was extended for 7 minutes to complete the polymerization of all chains. Thereafter the sample was cooled to 4°C. PCR products were isolated from the reaction mixture by two steps of chloroform extraction, DNA was than precipitated with isopropanol, dried in vacuum and dissolved in 50 µl of TE buffer.

Bacterium *E. coli K12*, JM109 (Yanisch-Perron et al., Gene 33 (1985) 103-119) was used in experiments of transformation and amplification of plasmids. Transformation and electroporation of *E. coli* were made according to previously described methods (Hanahan, D.J. Mol. Biol. 166, (1983) 557-580; Biorad Genepulser).

### Oligonucleotide Designing

The synthetic human proinsulin gene was derived from the published sequence of the human insulin gene (Bell et aL, Nature 284 (1980) 26-32). It should be pointed out that when synthesizing the gene, certain modifications can be made: it is possible to use various, in most cases preferred codons, to introduce additional functional DNA sequences and restriction sites in order to enable a ligation to expression vectors, to change one or more codons of a coding region in order to produce a structurally modified polypeptide, yet with substantially the same activity or utility.

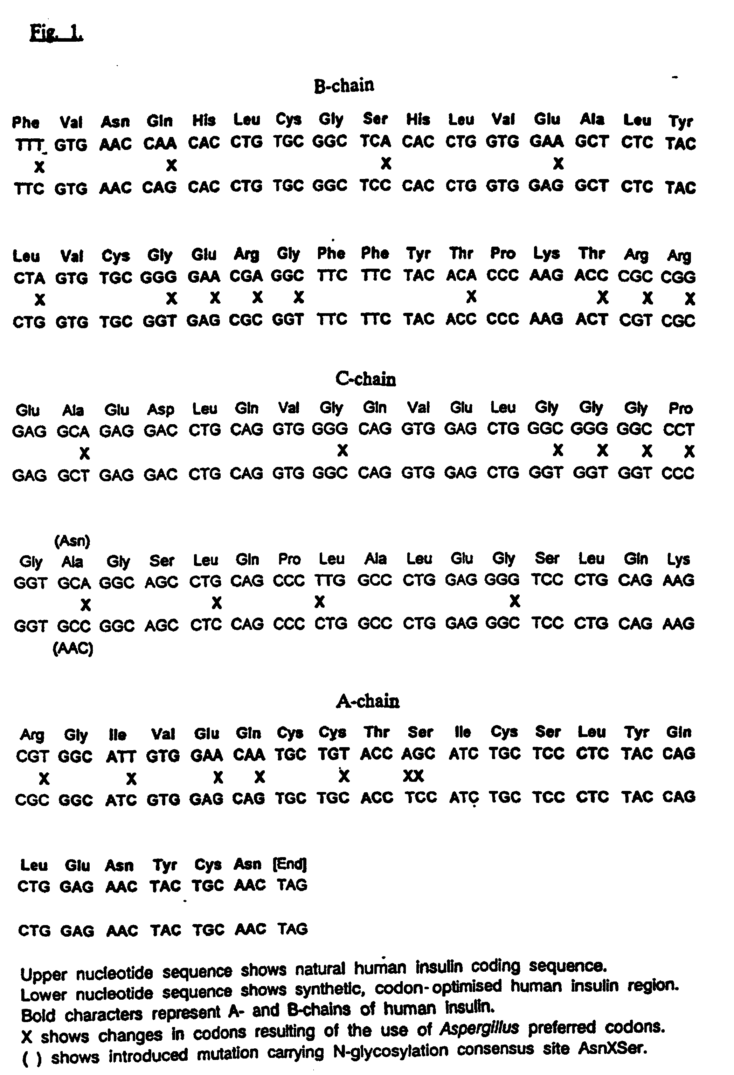

In the present invention, synthetic human proinsulin gene for expression in fungi was modified. The most frequently used codons of glucoamylase G1 gene of filamentous fungus *A. niger* were used, which is expressed in high quantities (Boel et al., EMBO J., 3, (1984) 1097-1102; Nunberg et al., Mol. Cell. Biol., 4 (1984) 2306-2315), and they were in most cases the same as human codons except in some regions where by choosing a preferred codon the oligonucleotide would become very rich in CG nucleotides, which could cause problems in sequencing and possibly even in PCR. Therefore some, mostly Gly codons were changed from GGC to GGT, which is the second preferred Gly codon in glucoamylase gene of filamentous fungus *A. niger* (Fig. 1).

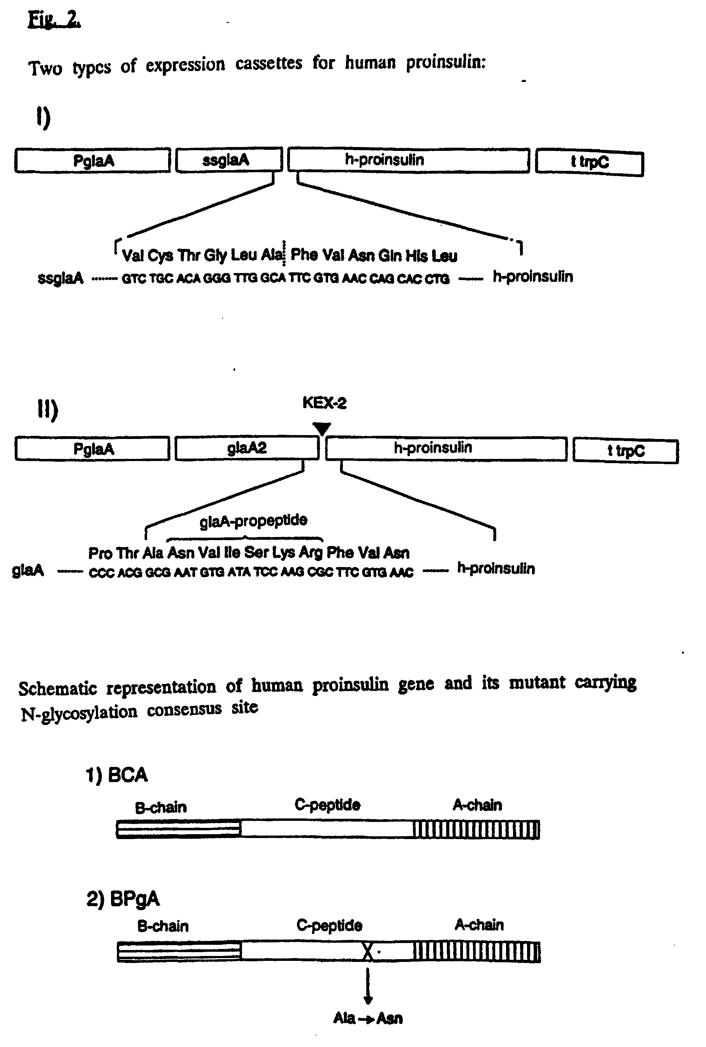

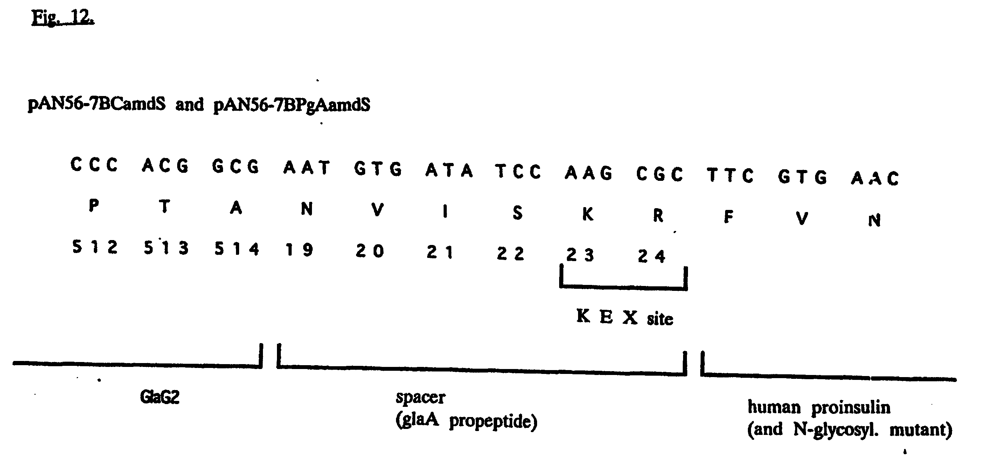

To the 5' end of synthetic proinsulin gene there were introduced additional DNA sequences encoding either 18 amino acids of glucoamylase gene (glaA) signal peptide or 6 amino acids of gene glaA propeptide containing Lys-Arg proteolytic cleavage site in order to make possible the construction of two types of expression cassettes. In the first case (I) the synthetic human proinsulin gene was regulated by glucoamylase promoter (p_{glaA}) and signal sequence (ss_{glaA}) of glucoamylase gene, whereas in the second case (II) proinsulin gene was fused with entire gene *glaA* of filamentous fungus *A. niger* via spacer peptide containing KEX-2-like protein processing signal under the control of p_{glaA} (Fig. 2 and Fig. 12).

Prepared was also a mutant of the synthetic human proinsulin gene carrying N-glycosylation consensus site for studying the influence upon the expression and secretion levels. Since C-peptide is not essential for insulin function because it is cleaved off in the secretion process, a mutation was introduced in the C-peptide coding region of the human proinsulin gene (BCA). A mutant (BPgA) carrying N-glycosylation consensus site AsnXSer was prepared by changing the codon GCC for Ala-C20 amino acid to codon AAC for Asn amino acid (Fig. 2).

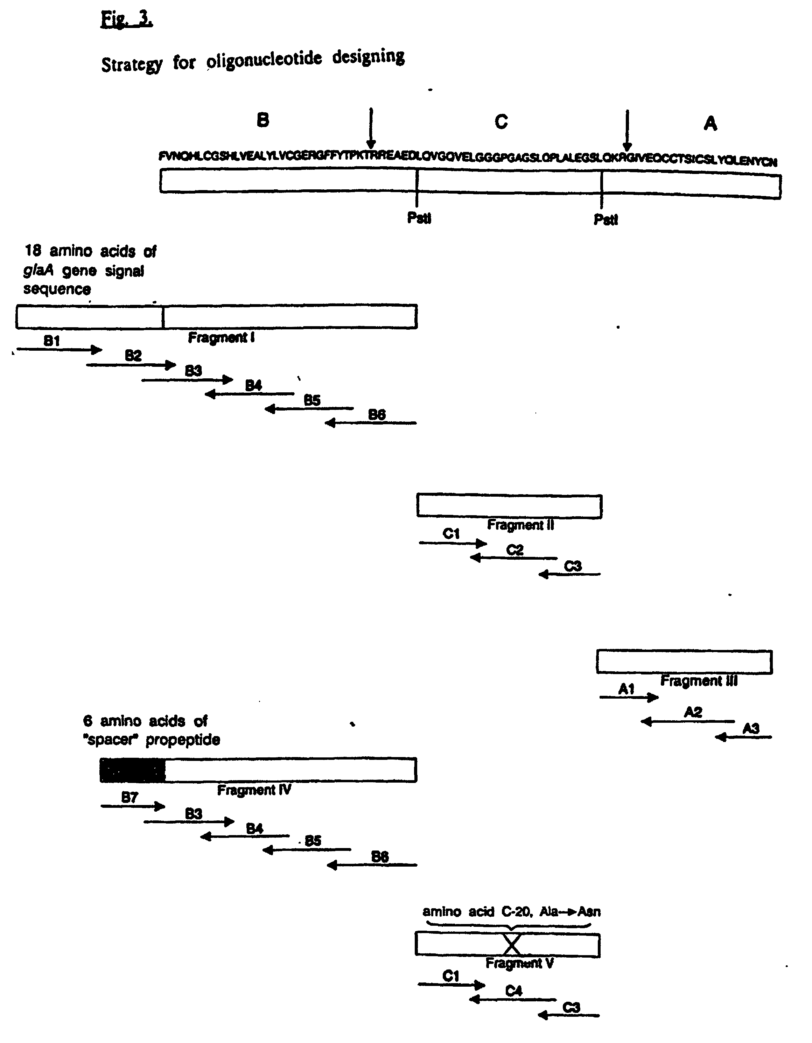

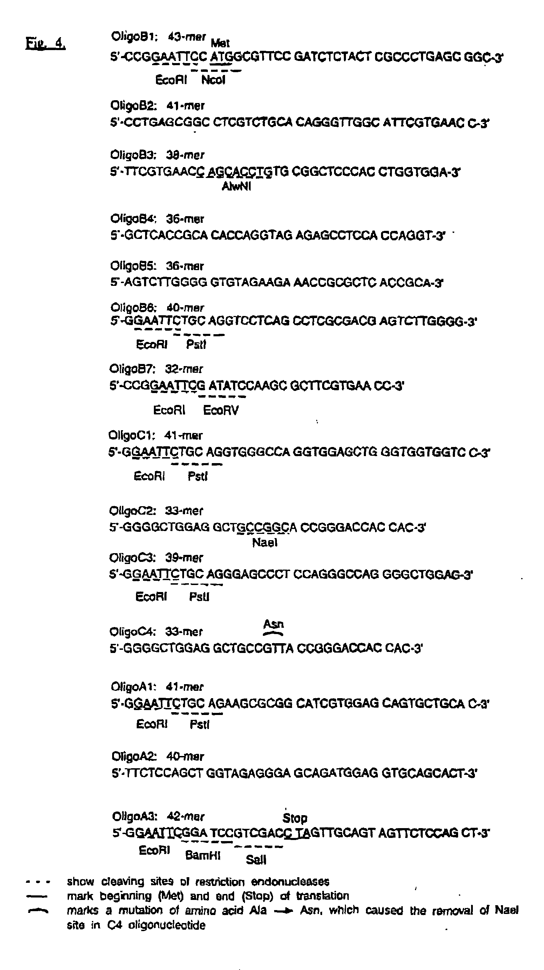

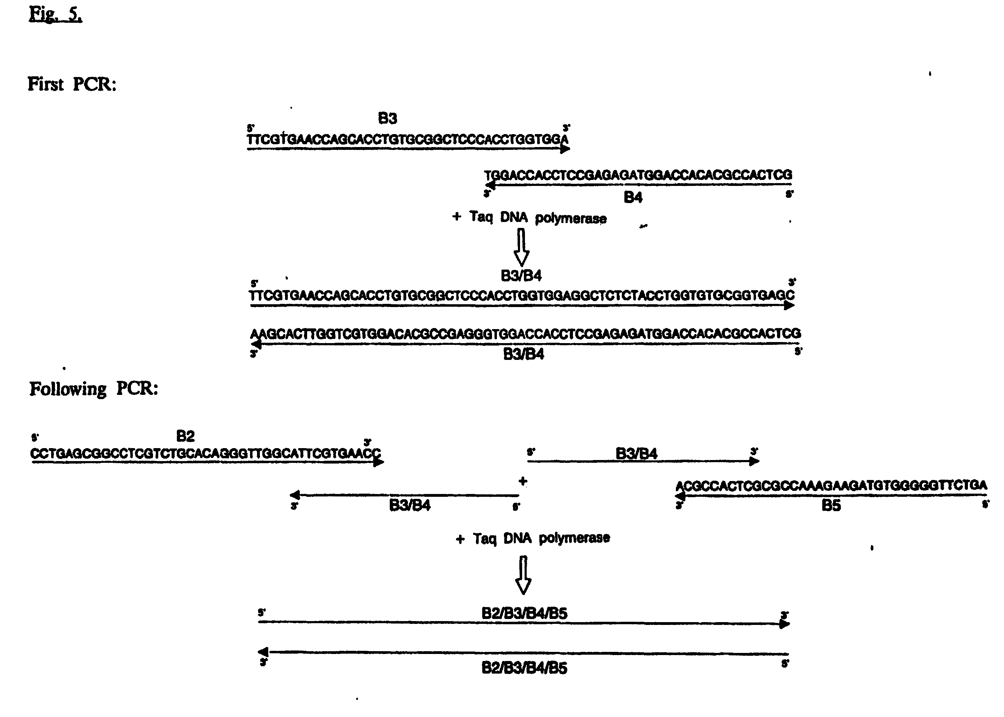

For the synthesis of the human proinsulin gene a new approach was introduced, wherein overlapping, complementary oligonucleotides in combination with PCR were used. The principle of the method was to use, during the PCR, two middle, overlapping, complementary oligonucleotides simultaneously as a template and primers, thus enabling TaqDNA polymerase to "enlarge" them, i.e. to elongate them during the first cycle. In the second cycle such "enlarged" oligonucleotides overlap with neighbouring 3' or 5' oligonucleotide, thus enabling TagDNA polymerase to "additionaly enlarge" already "enlarged" middle oligonucleotides. So TaqDNA polymerase actually fills the gaps between chemically synthesized, overlapping oligonucleotides and creates a DNA double helix. The synthesis of human proinsulin gene and its mutant comprising N-glycosylation consensus site was perfomed in fragments which were synthesized separately by PCR. The cleavage of genes in fragments was made possible by the presence of two cleavage sites for restriction enzym PstI in the C-peptide coding region (Fig. 3). Fourteen oligonucleotides were designed: ten oligonucleotides (B3, B4, B5, B6, C1, C2, C3, A1, A2 and A3) were deduced from the human proinsulin DNA encoding sequence with preferred codons for expression in filamentous fungus *A. niger*, two oligonucleotides (B1 and B2) were designed for the introduction of *glaA* gene signal sequence and additional restriction sites for making possible ligation into type I expression vector, one oligonucleotide (B7) was designed for the introduction of glaA (G2) gene sequence spacer region and additional restriction sites for making possible ligation with type II expression vector, and one oligonucleotide (C4) was designed for the introduction of mutation carrying N-glycosylation consensus site (Fig. 4).

### b) PCR synthesis of fragments I - V

### Fragments I and IV

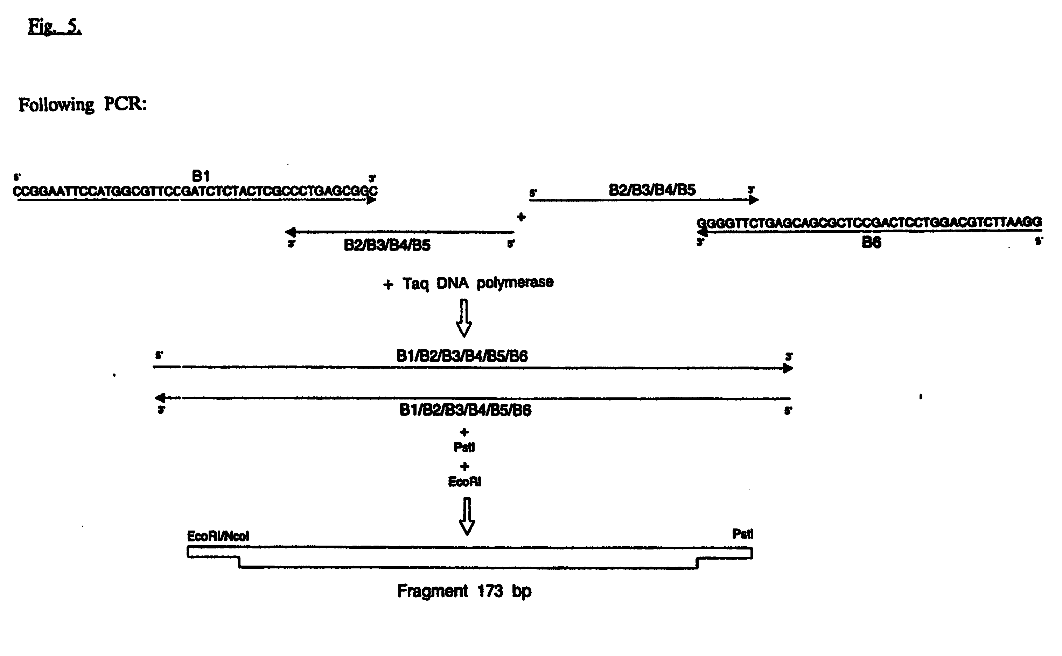

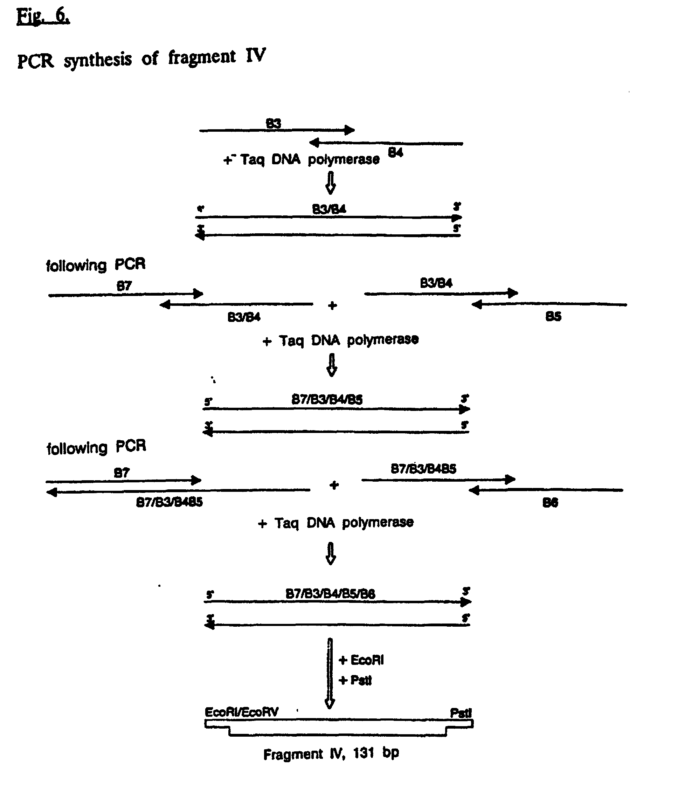

Fragments I and V were synthesized "step by step". In the first step of PCR, a reaction was performed with oligonucleotides B3 and B4, resulting in the product B3/B4. In the following "step", 1 µl of the product B3/B4 was mixed in PCR reaction mixture with: a) B2 and B5 oligonucleotides for fragment I, and b) B7 and B5 oligonucleotides for fragment IV. Products B2/B3/B4/B5 and B7/B3/B4/B5 were obtained. In the third "step", 1 µl of the product B2/B3/B4/B5 was used for PCR with oligonucleotides B1 and B6 for the fragment I, and 1 µl of the product B7/B3/B4/B5 was mixed in PCR reaction mixture with oligonucleotides B7 and B6 for the fragment IV. PCR products B1/B2/B3/B4/B5/B6 and B7/B3/B4/B5/B6 were obtained. Their size corresponded to the expected one, and the products were digested with appropriate restriction enzymes resulting in fragment I and fragment IV (Figs. 5 and 6).

### Fragments II, III and V

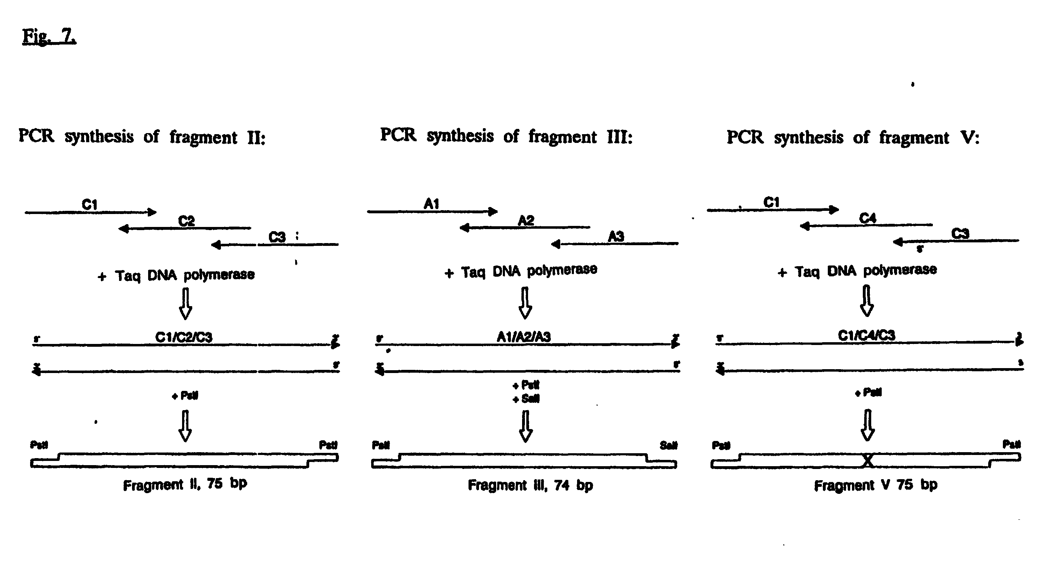

Fragments II, III and V were synthesized in one "step". PCR was performed with oligonucleotides: a) C1, C2, C3; b) A1, A2, A3; and c) C2, C4, C5. PCR products C1/C2/C3, A1/A2/A3 and C1/C4/C3 were of expected size and they were digested with appropriate restriction enzymes resulting in fragment II, fragment III and fragment V (Fig. 7).

### Cloning of the PCR-synthesized fragments into pUC19 vector and DNA sequence analysis

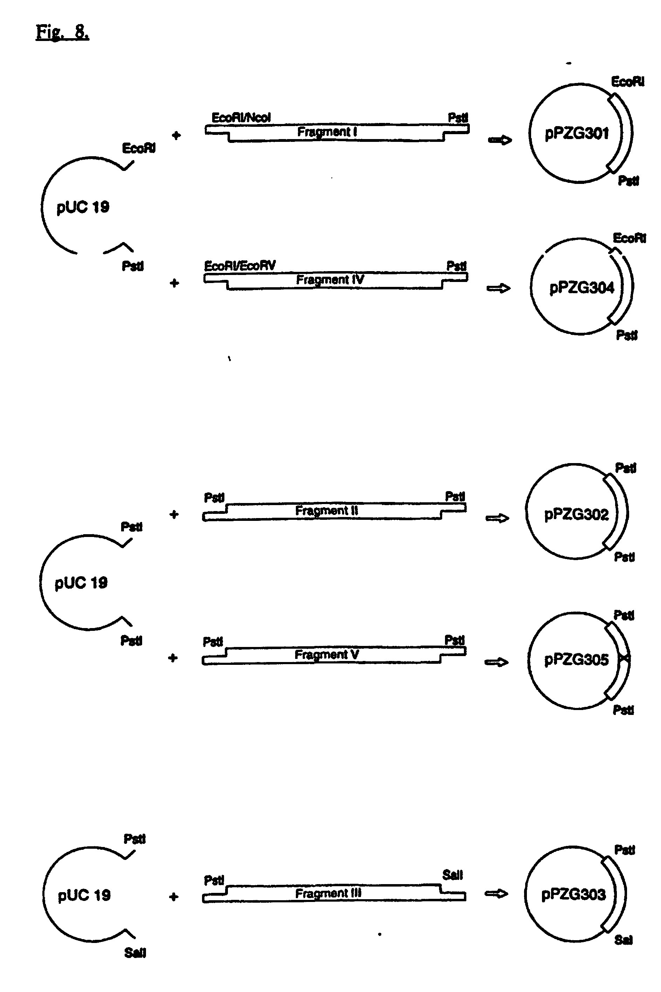

The plasmid pUC19 was digested with: a) PstI, b) PstI/EcoRI, and c) PstI/SalI restriction enzymes and ligated with appropriate fragments I-V, resulting in plasmids pPZG301, pPZG302, pPZG303, pPZG304 and pPZG305 (Fig. 8). With these plasmides the bacterium *E. coli* JM109 was transformed. From the obtained transformants pDNA was isolated by "mini preparation" and analyzed with restriction enzymes. *E. coli* transformants containing correct plasmids were grown, their pDNA was isolated by "QIAGEN typ 20" column and sequenced by "SEQUENASE® Version 2.0 kit". Vectors carrying a correctly synthesized and inserted sequence were used for further constructions.

### pPZG311 and nPZG312

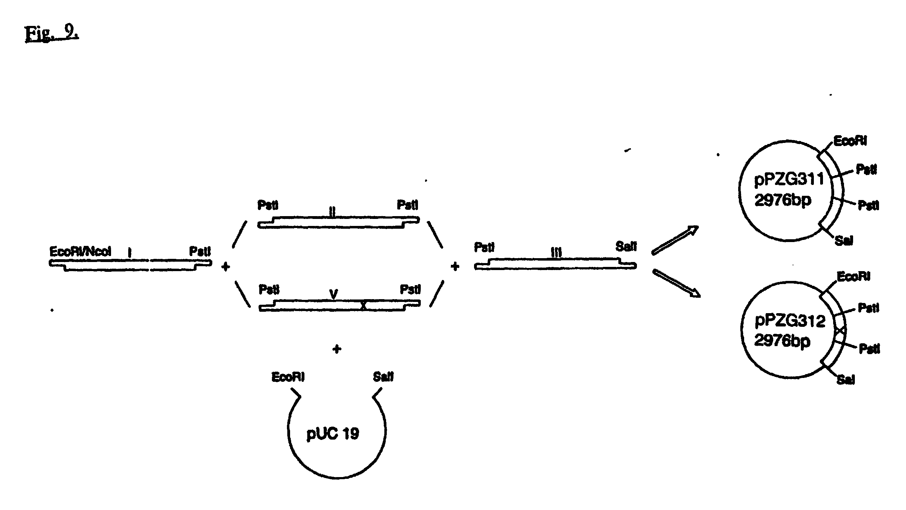

Vectors carrying the complete human proinsulin gene and mutant gene with N-glycosylation consensus site with the *glaA* gene signal sequence were constructed as follows: pPZG311 was constructed by a ligation of 173 bp EcoRI-PstI fragment isolated from pPZG301, 75 bp PstI-PstI fragment isolated from pPZG302, and 74 bp PstI-SalI fragment isolated from pPZG303 into EcoRI/SalI digested pUC19 vector. pPZG312 was constructed by a ligation of 173 bp EcoRI-PstI fragment isolated from pPZG301, 75 bp Pst-PstI fragment isolated from pPZG305, and 74 bp PstI-SalI fragment isolated from pPZG303 into EcoRI/SalI digested pUC19 vector (Fig. 9).

### Example 2

### Construction of filamentous fungal expression vector

### I. Expression cassette type I

### pAN52-7NotBCA and pAN52-7NotBPgA

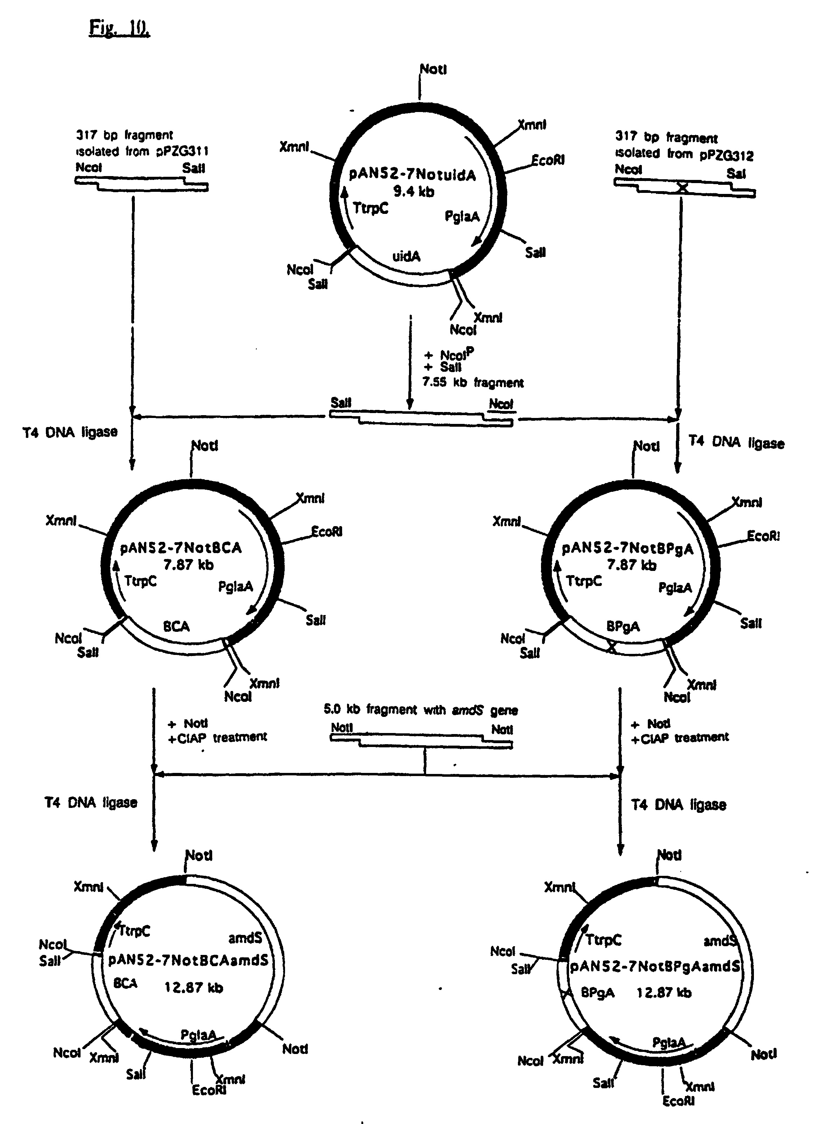

pAn52-7Not*uidA* (Verdoes et al., Gene (1994) in press) was digested partially with NcoI and with SalI. 7.55 kb SalI-NcoI fragment carrying *glaA* promoter and *trp*C gene termination region was isolated and ligated with 317 bp Nco-SalI fragment of plasmid pPZG311, resulting in plasmid pAN52-7NotBCA and 317 bp NcoI-SalI fragment of plasmid pPZG312, resulting in plasmid pAN52-7NotBPgA (Fig. 10).

### pAN52-7BCAamdS and pAN52-7BPgAamdS

5.0 kb NotI fragment carrying acetamydase (*amdS*) gene from *A. nidulans* was ligated into the NotI site of plasmids pAN52-7NotBCA and pAN52-7NotBPgA, resulting in plasmids pAN52-7BCAamdS and pAN52-7BPgAamdS (Fig. 10).

### II. Expression cassette type II

### pAN56-7BCAamdS and pAN56-7BPgAamdS

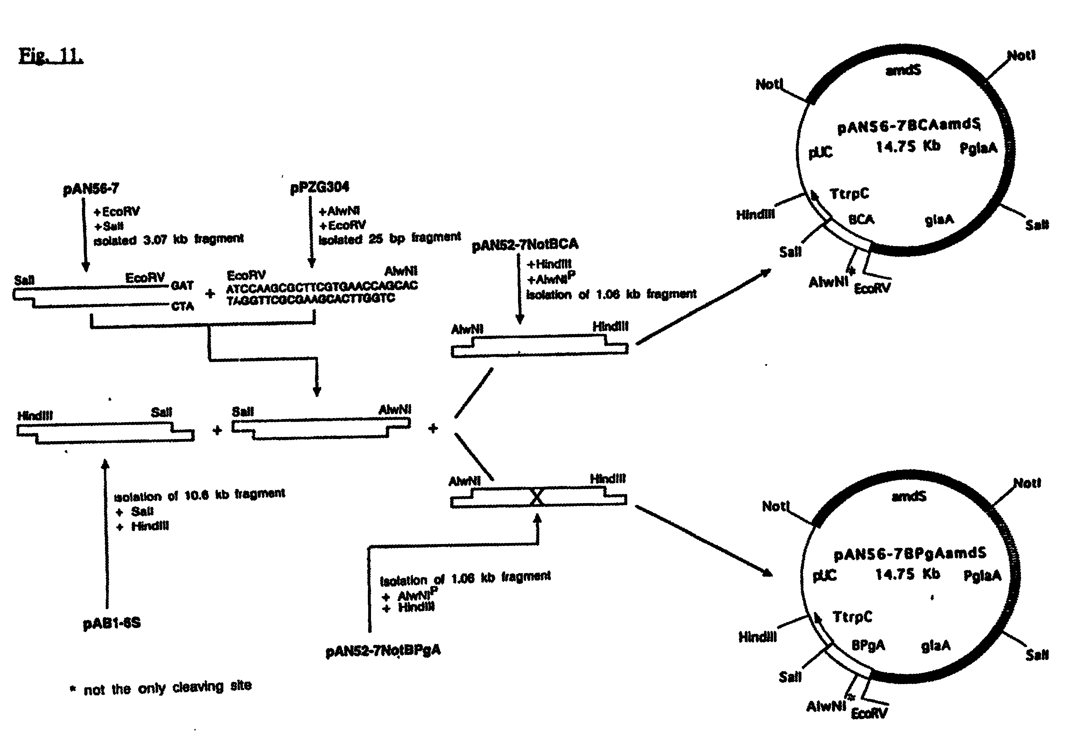

Vectors for fused glucoamylase-proinsulin protein and glucoamylase-proinsulin mutant with N-glycosylation consensus site were constructed as follows: plasmid pPZG304 was digested with EcoRV and AlwNI. Fragment EcoRV-AlwNI of 25 bp carrying sequence for 6 amino acids spacer was ligated with 3.07 kb SalI-EcoRV fragment from pAN56-7 carrying *glaA*(G2) gene, resulting in fragment SalI-AlwNI. Vector pAN56-7 was obtained from vector pAN56-4 (Broekhuijsen et al., J. Biotech., 31 (1993) 135-145), in which the *gpdA* promoter was replaced with 4.2 kb NotI-NcoI fragment from pAN52-7 carrying *glaA* promoter, and in which interleukin gene was removed, leaving EcoRV site upstream from GlaG2. Fragment AlwNI-SalI was then ligated with 10.6 kb HindIII-SalI fragment from pAB1-6S (Den Herder et al., MoL Gen. Genet. 233 (1992) 404-410) which has *glaA* promoter and *amdS* gene and with a) 1.06 kb AlwNI-HindIII fragment from pAN52-7BCA, resulting in plasmid pAN56-7BCAamdS, and b) 1.06 kb AlwNI-HindIII fragment from pAN52-7BPgA, resulting in vector pAN56-7BPgAamdS (Fig. 11). In the spacer there is a dibasic processing site Lys-Arg. The sequence around the fusion site of glucoamylase gene with human proinsulin gene (BCA) and proinsulin mutant (BPgA) by means of spacer region is shown in Fig. 12.

### Example 3

### Transformation of filamentous fungus Aspergillus niger

The transformation of filamentous fungus *Aspergillus niger* was carried out according to already described methods (Goosen et al., Curr. Genet. 11 (1987) 499-503; Kelly and Hynes, Embo J. 4 (1985) 475-479; Van Hartingsveldt et al., Mol. Gen. Genet. 206 (1987) 71-75).

Filamentous fungus *A. niger* N402 (Bos, C.J. et al., Curr. Genet 14 (1988) 437-443) was transformed with expression vectors pAN52-7BCAamdS, pAN52-7BPgAamdS, pAN56-7BCAamdS and pAN56-7BPgAamdS. Colonies were selected on culture mediums with acetamide or acrylamide as the sole carbon and nitrogen source. Starting from single spores, a further analysis of transformants was performed.

### Example 4

### Secretion of human insulin into the culture medium

### 1. Culturing of A. niger transformants carrying human proinsulin gene and mutant gene with N-glycosylation consensus site

About 10⁸ spores of selected transformants were inoculated into 500 ml Erlenmeyer flasks containing 100 ml of liquid culture medium containing: 70 mM NaNO₃, 7 mM KCl, 11 mM KH₂PO₄, 2 mM MgSO₄, 4% dextrin, trace elements (1:1000; 76 mM ZnSO₄, 178 mM H₃BO₃, 25 mM MnCl₂, 18 mM FeSO₄, 7.1 mM CoCl₂, 6.4 mM CuSO₄, 6.2 mM Na₂MoO₄, 174 mM EDTA) and vitamins (1:1000; 100 mg/L of thiamine, 100 mg/L of riboflavine, 100 mg/L of nicotinamide, 50 mg/L of pyridoxin, 10 mg/L of panthotenic acid, 0.2 mg/L of biotin). Transformants were grown at 32°C for 36 hours. Samples were analysed on insulin presence.

### 2. Expression of insulin

Expression of immunoreactive human insulin was demonstrated by "Pharmacia INSULIN RIA 100" kit, Kabi Pharmacia Diagnostics. Samples of transformants cultured by fermentation were filtrated, filtrates were neutralized, diluted if necessary and then submitted to RIA (radioimmunoassay) determination (Livessy et al., Clin. Biochem. 13 (1980) 151-55). The expression level of immunoreactive human insulin (mU/L) in *A. niger* transformants is shown in Table 1.

**Table 1**

| Expression level of immunoreactive human insulin in various *A. niger* transformants: | | |
|---|---|---|
| Transformant | Plasmid | Immunoreactive insulin (mU/L) |
| *A. niger* 7650 [NCAIM(P)F 001215] | PAN52-7BCA*amdS* | 0 |
| *A. niger* 7651 [NCAIM (P) F 001216] | PAN52-7BPgA*amdS* | 2 |
| *A. niger* 7649 [NCAIM (P) F 001214] | PAN56-7BCA*amdS* | 56 |
| *A. niger* 7638 [NCAIM (P) F 001213] | PAN56-7BPgA*amdS* | 776 |

Transformants *A. niger* 7650, *A. niger* 7651, *A. niger* 7649 and *A. niger* 7638 were deposited in "National Collection of Agricultural and Industrial Microorganisms, H-1502 Budapest, P.O.B. 53, Hungary, on July 8, 1994 under accession numbers NCAIM (P) F 001215, NCAIM (P) F 001216, NCAIM (P) F 001214 and NCAIM (P) F 001213 and in accordance with the Budapest Treaty on the International Recognition of the Deposit of Microorganisms for the Purposes of Patent Procedure, Statement in the Case of an Original Deposit, pursuant to Rule 6.1.

From the Table it is evident that there is a distinct difference in the expression of the transformants containing various vectors. By far the best result are shown by a transformant obtained by transformation with a vector having human proinsulin gene carrying N-glycosylation consensus site fused with glucoamylase protein.

## Claims

1. DNA sequence comprising a sequence encoding insulin precursor of formula B-Pg-A, wherein B and A represent B- and A-chains of human insulin, respectively, Pg represents modified C-peptide or any number of amino acids comprising at least one N-glycosylation consensus site, and Pg links B- and A-chains by proteolytic processing signals or specific chemical cleavage sites.

2. DNA sequence comprising a sequence encoding insulin precursor of formula B(29)-Pg-A, wherein B(29) represents amino acids 1-29 of B-chain of human insulin, A represents A-chain of human insulin, Pg represents modified C-peptide or any number of amino acids comprising at least one N-glycosylation consensus site and Pg links B- and A-chains by proteolytic processing signals or specific chemical cleavage sites.

3. DNA sequence according to claim 1 or 2 with preferred codons for expression in particular hosts.

4. DNA sequence according to anyone of claims 1 to 3 fused with a protective protein gene by means of a spacer region carrying a proteolytic processing signal or specific chemical cleavage site.

5. Transformed cells of a suitable host comprising a DNA sequence according to anyone of claims 1 to 4.

6. Transformed fungal cells comprising a DNA sequence according to anyone of claims 1 to 4.

7. Transformed *Aspergillus sp.* fungal cells comprising a DNA sequence according to anyone of claims 1 to 4.

8. A process for the preparation of insulin by culturing suitable cells, preferably fungal cells, transformed with a vector comprising a DNA sequence according to anyone of claims 1 to 4.

## Patentansprüche

1. DNA-Sequenz enthaltend eine Insulinvorstufe kodierende Sequenz der Formel B-Pg-A, worin B und A B- bzw. A-Ketten des menschlichen Insulins darstellen, Pg ein modifiziertes C-Peptid oder irgendeine Zahl von Aminosäuren, die wenigstens eine N-Glykosylierungskonsensusstelle umfassen, darstellt und Pg die B- und A-Ketten durch proteolytische Prozessierungssignale oder spezifische chemische Spaltungsstellen verbindet.

2. DNA-Sequenz enthaltend eine Insulinvorstufe kodierende Sequenz der Formel B(29)-Pg-A, worin B(29) Aminosäuren 1-29 der B-Kette des menschlichen Insulins darstellt, A eine A-Kette des menschlichen Insulins darstellt, Pg ein modifizertes C-Peptid oder irgendeine Zahl von Aminosäuren, die wenigstens eine N-Glykosylierungskonsensusstelle umfassen, darstellt und Pg die B- und A-Ketten durch proteolytische Prozessierungssignale oder spezifische chemische Spaltungsstellen verbindet.

3. DNA-Sequenz gemäss Anspruch 1 oder 2 mit bevorzugten Kodonen für die Expression in bestimmten Wirten.

4. DNA-Sequenz gemäss irgendeinem der Ansprüche 1 bis 3, fusiert mit einem Schutzproteingen mittels einer Abstandshalterregion, die ein proteolytisches Prozessierungssignal oder eine spezifische chemische Spaltungsstelle trägt.

5. Transformierte Zellen eines geeigneten Wirtes, die eine DNA-Sequenz gemäss irgendeinem der Ansprüche 1 bis 4 umfassen.

6. Transformierte Pilzzellen enthaltend eine DNA-Sequenz gemäss irgendeinem der Ansprüche 1 bis 4.

7. Transformierte *Aspregillus sp.* Pilzzellen enthaltend eine DNA-Sequenz gemäss irgendeinem der Ansprüche 1 bis 4.

8. Verfahren zur Herstellung von Insulin mittels Vermehrung von geeigneten Zellen, vorzugsweise Pilzzellen, transformiert mit einem Vektor, der eine DNA-Sequenz gemäss irgendeinem der Ansprüche 1 bis 4 umfasst.

## Revendications

1. Séquence d'ADN comprenant une séquence codant pour un précurseur d'insuline de formule B-Pg-A, dans laquelle B et A représentent respectivement des chaînes B et A d'insuline humaine, Pg représente un peptide C modifié ou un nombre quelconque d'acides aminés comprenant au moins un site consensus de N-glycosylation, et Pg lie les chaînes B et A par des signaux de traitement protéolytique ou des sites de clivage chimique spécifiques.

2. Séquence d'ADN comprenant une séquence codant pour un précurseur d'insuline de formule B(29)-Pg-A, dans laquelle B(29) représente les acides aminés 1-29 de la chaîne B d'insuline humaine, A représente la chaîne A d'insuline humaine, Pg représente un peptide C modifié ou un nombre quelconque d'acides aminés comprenant au moins un site consensus de N-glycosylation et Pg lie les chaînes B et A par des signaux de traitement protéolytique ou des sites de clivage chimique spécifiques.

3. Séquence d'ADN suivant l'une ou l'autre des revendications 1 et 2, avec des codons avantageux pour l'expression dans des hôtes particuliers.

4. Séquence d'ADN suivant l'une quelconque des revendications 1 à 3, fusionnée à un gène de protéine de protection au moyen d'une région d'espacement comportant un signal de traitement protéolytique ou site de clivage chimique spécifique.

5. Cellules transformées d'un hôte approprié comprenant une séquence d'ADN suivant l'une quelconque des revendications 1 à 4.

6. Cellules fongiques transformées comprenant une séquence d'ADN suivant l'une quelconque des revendications 1 à 4.

7. Cellules fongiques d'Aspergillus sp. transformées comprenant une séquence d'ADN suivant l'une quelconque des revendications 1 à 4.

8. Procédé de préparation d'insuline par la culture de cellules appropriées, avantageusement des cellules fongiques transformées avec un vecteur comprenant une séquence d'ADN suivant l'une quelconque des revendications 1 à 4.
